Europäisches Patentamt

European Patent Office

Office européen des brevets

(11) Publication number: 0 682 942 A1

# EUROPEAN PATENT APPLICATION
## published in accordance with Art. 158(3) EPC

(21) Application number: 94904738.5

(22) Date of filing: 19.01.94

(86) International application number: PCT/JP94/00066

(87) International publication number: WO 94/16682 (04.08.94 94/18)

(51) Int. Cl.⁶: **A61K 9/107**, A61K 9/70, A61K 47/06

(30) Priority: **21.01.93 JP 26260/93**

(43) Date of publication of application: **22.11.95 Bulletin 95/47**

(84) Designated Contracting States: **AT BE CH DE DK ES FR GB GR IE IT LI LU NL PT SE**

(71) Applicant: **YAMANOUCHI PHARMACEUTICAL CO. LTD.**
**No. 3-11 Nihonbashi-Honcho, 2-chome Chuo-ku Tokyo 103 (JP)**

(72) Inventor: **KATSUMA, Masataka**
**3-1-201, Surugadai 1-chome Fujieda-shi Shizuoka 426 (JP)**
Inventor: **KAWAI, Hitoshi**
**180-1, Ozumi Yaizu-shi Shizuoka 425 (JP)**
Inventor: **SAITO, Katsumi**
**180-1, Ozumi Yaizu-shi Shizuoka 425 (JP)**
Inventor: **SUZUKI, Naoko**
**2541-1, Wakamatsu-cho Simada-shi Shizuoka 427 (JP)**
Inventor: **KONNO, Yutaka**
**728-9, Gokahorinouchi Yaizu-shi Shizuoka 425 (JP)**

(74) Representative: **Geering, Keith Edwin et al REDDIE & GROSE 16 Theobalds Road London WC1X 8PL (GB)**

(54) NOVEL, PERCUTANEOUSLY ABSORBABLE PREPARATION.

(57) A medicinal composition for external use of O/W emulsion type which comprises (a) a physiologically active substance wholly or partially soluble in aqueous ethanol, (b) a monoterpene difficulty soluble in aqueous ethanol or a mixture thereof with a monoterpene soluble in aqueous ethanol, (c) a nonionic surfactant, and (d) aqueous ethanol. The invention provides a highly safe, percutaneously absorbable preparation which can allow desirable skin penetration of a medicine and does not irritate the skin.

EP 0 682 942 A1

TECHNICAL FIELD

This invention relates to a novel transdermal absorption preparation. More particularly, it relates to a transdermal absorption preparation which can efficiently deliver a physiologically active substance of interest into the living body.

BACKGROUND ART

As a technique which enables systemic treatment by transdermal administration, the transdermal therapeutic system (TTS) has been proposed on the basis of the idea of pharmacokinetics or drug delivery system and on the advance in drug preparation techniques. The application of the TTS to various drugs has been attempted because of its advantages in that commencement and termination of drug administration can be made easily, prolonged effects can be obtained, side effects can be reduced and inactivation by first-pass in the liver can be avoided, and the TTS for scopolamine, nitroglycerin, clonidine and isosorbide dinitrate is already on the market.

It is well known that epidermal tissues of animals are generally have a barrier mechanism which can inhibit invasion of foreign matters and that the transdermal absorption of a drug is determined by mutual interaction among the drug, base to be used, and the skin. In consequence, the design of the preparation for the TTS is attained by overcoming several problems related to the transdermal absorption of the drug used, wherein the most important subjects from a viewpoint of expressing accurate drug effect are to increase penetration rate of the drug at the horny layer penetration step which is a rate-determining step of the transdermal absorption and to design a safe system which does not irritate the skin without spoiling the thus increased transdermal penetration of the drug.

On the basis of such background art, studies have been made on various transdermal absorption-enhancing agents. In recent years, natural terpenes have been noted as transdermal absorption-enhancing agents which are highly safe, have excellent effects and can be used as substitutes for the known synthetic transdermal absorption-enhancing agents such as dimethyl sulfoxide (DMSO), Eison (trade name, manufactured by Nelson), and the like and have been used for various drugs. For example, it is known that limonene, one of monocyclic monoterpenes shows significant effects on the enhancement of the transdermal absorption of fat-soluble drugs such as indometacin, ketoprofen and the like (*Drug Design and Delivery*, 4, 313 (1989)). It has also been reported that ethanol can serve as a transdermal absorption-enhancing auxiliary which improves absorption-enhancing effect on water soluble drugs, when added as a solvent to a system in which *d*-limonene or the like is used as a transdermal absorption-enhancing agent (Abstract of Papers, the 6th Annual Meeting of the Pharmaceutical Society of Japan, pp. 40 - 41, 1990), but its transdermal absorption-enhancing effect is not enough. This reference also reported that the skin irritation was observed when large amounts of limonene and ethanol were added.

On the other hand, it has been reported that transdermal absorption of drugs can be improved when limonene used as a transdermal absorption-enhancing agent is dispersed in water by the action of a nonionic surface active agent, thus revealing that the transdermal drug absorption-enhancing effect of terpenes can be improved by the use of emulsion instead of solvent (cf. unexamined published Japanese Patent Application (*Kokai*) No. 3-127744).

However, since this technique cannot sufficiently improve the effects of terpenes (e.g., limonene) to enhance the transdermal drug absorption, great concern has been directed toward the development of a TTS preparation of an emulsion system containing a terpene as the oil phase, which is safe and can exert practical transdermal drug absorption-enhancing effect.

DISCLOSURE OF THE INVENTION

In view of such a technical level, the inventors of the present invention have conducted intensive studies with the aim of developing a pharmaceutical preparation which can be constructed as a highly safe and administration-controllable practical TTS system in which the transdermal drug absorption-enhancing property of highly safe terpenes is effectively exerted in an emulsion system.

As a result, it was found quite unexpectedly that the transdermal drug absorption-enhancing property of terpenes can be improved markedly without causing irritation to the skin, when ethanol, which is generally considered to be a solvent that spoils the transdermal absorption-enhancing property by dissolving oil of the emulsion and therefore destroying the emulsion, simultaneously showing irritation to the skin, is added at a specified ratio of the water content in the presence of a specified nonionic surface active agent, hence resulting in the accomplishment of the present invention.

The following describes the present invention in detail.

The emulsion is generally divided into an oil-in-water (O/W) emulsion type in which oil drops (inner oil layer) are dispersed in water (outer water layer), a water-in-oil (W/O) emulsion type in which water drops (inner water layer) are dispersed in oil (outer oil layer) and a complex (W/O/W or O/W/O) emulsion type in which these emulsion types are re-dispersed in a third layer component. The O/W emulsion type is preferred in the present invention from the viewpoint of the solubility of drugs in the combination system of drugs, terpenes, nonionic surface active agents and the like and of the physical properties of terpenes as the base such as the skin irritation and the like.

According to the transdermal absorption preparation of the present invention, any monoterpenes or essential oils containing monoterpenes as main components may be used as the transdermal absorption-enhancing agent as long as they are hardly soluble in aqueous ethanol used as the base, and examples thereof include monoterpenes such as limonene, caren, pinene, menthane, terpinen, terpinolene, menthone, carvone, cineole, pulegone, *d*-camphor, linalool, citral, nerol, geraniol, citronellol and the like and essential oils containing these monoterpenes as the main component, such as Orange oil (limonene as the main component), Eucalyptus oil (cineole as the main component), Turpentine oil (pinene as the main component), Spearmint oil (*l*-carvone as the main component), Lemon oil (limonene as the main component), Mentha oil (*l*-menthol as the main component) and the like. Of these compounds, essential oils containing a cyclic monoterpene as main component and cyclic monoterpenes belonging to hydrocarbons are preferred, more preferably limonene, Orange oil, Eucalyptus oil, Turpentine oil, Lemon oil and Spearmint oil and most preferably Eucalyptus oil. As a matter of course, these compounds may be used in combination. Since these compounds are composing element of the inner oil layer in the transdermal absorption preparation of the emulsion type of the present invention, their mixing amount should be adjusted depending on the composition ratio in the outer water layer which comprises aqueous ethanol, but it may preferably be in the range of from 1 to 50% by weight, more preferably from 1 to 10% by weight. When the amount is smaller than 1%, a sufficient transdermal absorption-enhancing effect cannot be obtained, because the aforementioned terpenes are dissolved in the outer water layer and thus the emulsion is not formed. On the other hand, the amount if larger than 50% would produce no proportionally greater effect, but would rather increase irritation to the skin. Though the term "hardly soluble monoterpenes" as used herein means that they are insoluble in the aqueous ethanol to be used, any terpene which is soluble in aqueous ethanol can also be used as the hardly soluble monoterpene of the present invention, because it can constitute the inner oil layer when used in an amount larger than its solubility.

Examples of such a case include Eucalyptus oil, Spearmint oil, Mentha oil, cineole and the like. In this connection, according to the preparation of the present invention, addition of the aforementioned monoterpenes hardly soluble in aqueous ethanol together with the monoterpenes soluble in aqueous ethanol is effective in further improving the transdermal absorption-enhancing effect.

Examples of the useful monoterpenes which are soluble in aqueous ethanol include cyclic monoterpenes such as *l*-menthol, terpineol, carvone, borneol, menthone, cineole, pulegone, *d*-camphor, Spearmint oil, Mentha oil and the like and straight chain monoterpenes such as linalool, citral, geraniol, nerol, citronellol and the like. Preferably, *l*-menthol, terpineol, borneol, linalool and Mentha oil may be used. More preferably, *l*-menthol and Mentha oil may be used. As a matter of course, their mixing amount should be adjusted based on the amount of ethanol used in the aqueous ethanol, but the amount may be in the range of from 0.1 to 10% by weight, preferably from 0.5 to 5%. The reason is that the absorption-enhancing effect of the combined use could not be obtained when the mixing amount is less than 0.1%, and separation of emulsion could occur when the amount is more than 10%.

Preferred examples of the combination of monoterpenes hardly soluble in aqueous ethanol with monoterpenes soluble therein include a combination of Orange oil with Mentha oil, a combination of Orange oil with *l*-menthol and a combination of Orange oil with linalool, as well as a combination of Eucalyptus oil with Mentha oil and a combination of Eucalyptus oil with *l*-menthol.

The transdermal absorption preparation of the present invention is characterized in that the aforementioned terpenes are emulsified in aqueous ethanol, wherein the ethanol contained in the outer water layer is an essential composing element for sufficiently generating the absorption-enhancing effect of the aforementioned terpenes. Another effect of using an aqueous ethanol system as the outer water layer is that the solubility of drugs is improved so that the range of applicable drugs can be broadened. The aqueous ethanol to be used in the present invention is not particularly limited, provided that ethanol contains at least 5% of aqueous components. In addition to water as a solvent, the aqueous components may further include pharmaceutically acceptable known components such as a buffer, a stabilizing agent, an antioxidant, a pH regulating agent and the like. Ethanol may be used within the range of generally from 1 to 70% by weight, preferably from 5 to 50% by weight, based on the total components. The amount if smaller than 1% would not exhibit the sufficient absorption-enhancing effect of the aforementioned terpenes, and if larger than 70% would cause not only a considerable decrease in the absorption-enhancing effect due to disintegration of emulsion (cf. Test Example 2 which will be described later) but also possibility of inducing irritation to the skin.

When Eucalyptus oil as one of the monoterpenes which are hardly soluble in aqueous ethanol is used in an amount of 5% by weight, it is partially dissolved when the ethanol content exceeds 55% by weight. It was confirmed, however, that the emulsion can be formed again after application to the skin.

It was also confirmed that the absorption-enhancing effect of Eucalyptus oil can be obtained sufficiently even in the just described case.

The emulsifying agent to be used in the transdermal absorption preparation of the present invention is not particularly limited, provided that it can emulsify the aforementioned terpenes in the aqueous ethanol. In any case, it is preferable to use a nonionic surface active agent rather than ionic surface active agents which are generally considered to be irritable to the skin.

Illustrative examples of the emulsifying agent include hydrophilic nonionic surface active agents such as polyoxyethylene phenyl ether, polyoxyethylene alkyl ether, polyoxyethylene alkyl sorbitan fatty acid ester, polyethylene glycol fatty acid ester, polyoxyethylene polyoxypropylene alkyl ether, polyoxyethylene castor oil, polyoxyethylene hardened castor oil and the like and hydrophobic nonionic surface active agents which may optionally be used in combination with the above hydrophilic agents, such as sorbitan fatty acid ester, glycerol fatty acid ester, glycerol polyfatty acid ester and the like. Since the emulsion-type transdermal absorption preparation of the present invention is characterized in that the outer water layer contains ethanol, the emulsification becomes difficult depending on the content of ethanol. Accordingly, it is desirable to select and control a surface active agent so that the roughly calculated HLB (hydrophile lipophile balance) value becomes to 9.5 or more. When intensive studies were conducted on the emulsifying agent to be used in the present invention, it was found that polyoxyethylene hardened castor oil having an HLB value of 11 or more is desirable because it can form excellent emulsion even when the ethanol content is 30% or more. Alternatively, the HLB value may be adjusted to 11 or more by mixing this oil with other emulsifying agent. It is necessary to adjust mixing amount of the emulsifying agent depending on the composition ratio of the outer water layer having aqueous ethanol, similar to the case of the aforementioned terpenes. However, it may be within the range of preferably from 0.1 to 20% by weight, more preferably from 0.25 to 10% by weight. The reason is that the amount if smaller than 0.1% would not result in the formation of emulsion, and if larger than 20% would produce no proportionally greater effects in terms of changes in the emulsification and improvement in the absorption property. In addition, disintegration of emulsion could occur depending on the type of the surface active agent.

The physiologically active substance to be used in the transdermal absorption preparation of the present invention can optionally be selected from known physiologically active substances which are soluble or partially soluble in the aqueous ethanol used as the base. Though not particularly limited, illustrative examples of such physiologically active substances include: serotonin $5HT_3$ receptor antagonists such as (-)-(R)-5-[(1-methyl-1H-indol-3-yl)carbonyl]-4,5,6,7-tetrahydro-1H-benzimidazole (ibusetron) and salts thereof (e.g., ibusetron hydrochloride), ondansetron, granisetron and the like; non-steroidal anti-inflammatory drugs such as indometacin, ibuprofen, ibufenac, alclofenac, diclofenac, mefenamic acid, flurbiprofen, flufenamic acid, ketoprofen, phenylbutazone, methyl salicylate and the like;
steroidal anti-inflammatory drugs such as cortisone, hydrocortisone, prednisolone, dexamethasone, betamethasone dipropionate, betamethasone valerate, prednisolone, triamcinolone, fluocinolone acetonide and the like;
diuretics such as bendroflumethiazide, polythiazide, methyclothiazide, trichlormethiazide, cyclopenthiazide, pentyl hydrochlorothiazide, hydrochlorothiazide, bumetanide and the like;
antipsychotics such as emonapride, diazepam, nitrazepam, flunitrazepam, lorazepam, prazepam, fludiazepam, clonazepam, chlorpromazine, reserpine, trifluperidol, haloperidol, moperone and the like;
hypnotics such as barbital, thiopental, phenobarbital, cyclobarbital and the like;
antiepileptics such as ethosuximide, sodium valproate, acetazolamide, meprobamate and the like;
antiparkinsonism drugs such as chlorzoxazone, levodopa and the like;
antiemetics such as metoclopramide, metoclopramide hydrochloride and the like;
hormones such as insulin, testosterone, methyl testosterone, progesterone, estradiol and the like;
sedatives such as morphine, aspirin, codeine, acetanilide, aminopyrine and the like;
sulfoamides such as sulfanilamide, sulfamonomethoxine, sulfamethizole and the like;
coronary vasodilators such as nitroglycerin, isosorbide dinitrate, pentaerythrityl tetranitrate, propatylnitrate, dipyridamole, papaverine hydrochloride and the like;
antiarrhythmic drugs such as ajimalin, pindolol, propranolol, quinidine and the like;

EP 0 682 942 A1

cardiotonics such as caffeine, digoxin, digitoxin, amrinone, milrinone and the like;

calcium antagonists such as nicardipine hydrochloride, diltiazem hydrochloride, nivadipine, nifedipine, nitrendipine, nisoldipine, nimodipine, niludipine and the like;

antihistaminics such as diphenhydramine hydrochloride, carbinoxamine, diphenylpyrallin, phenbenzamine, chlorpheniramine maleate, brompheniramine maleate, diphenylimidazol, clemizole and the like;

antibiotics such as tetracycline, oxytetracycline, metacycline, doxycycline, minocycline, chloramphenicols, erythromycins, lincomycin, penicillin G, clindamycin, kanamycin, fradiomycin, streptomycin, gentamicin and the like;

local anesthetics such as procaine hydrochloride, tetracaine hydrochloride, dibucaine hydrochloride, lidocaine hydrochloride, piperocaine acetate, benzocaine and the like; antifungal agents such as tolnaftate, griseofulvin, diamethazol hydrochloride, aureothricin, trichomycin, pyrrolnitrin, 5-fluorocytosine, benzalkonium chloride, acetophenylamine, nitrofurazone, pentamycin and the like; anti-malignant tumor drugs such as 5-fluorouracil, uracil, cytarabine, floxuridine, busulfan, actinomycin, bleomycin, mitomycin and the like; antidiabetics such as glibenclamide and the like;

potassium channel opening agents such as 2-(3,4-dihydro-2,2-dimethyl-6-nitro-2H-1,4-benzoxazin-4-yl)-pyridine N-oxide and salts thereof, cromakalim, lemakalim and the like;

drugs for gout treatment use such as allopurinol, colchicine, benzbromarone and the like;

antiallergic agents such as ketotifen fumarate, sodium cromoglicate, amlexanox and the like;

antihypertensive drugs such as clonidine, guanethidine sulfate, amosulalol hydrochloride, alacepril, delapril hydrochloride, enalapril maleate and the like;

drugs for central nervous system such as indeloxazine hydrochloride, tiapride hydrochloride, bifemelane hydrochloride and the like;

skeletal muscle relaxants such as dantrolene sodium and the like;

antispastics such as eperisone hydrochloride, tizanidine hydrochloride, butylscopolamine, atropine methylbromide and the like;

drugs for hyperlipemia use such as simvastatin, pravastatin sodium and the like;

bronchodilators such as formoterol fumarate, salbutamol sulfate, procaterol hydrochloride and the like; and α-adrenaline receptor blocking drugs such as tamusulosin hydrochloride, prazosin and the like.

Of these various physiologically active substances, ibusetron hydrochloride, tamusulosin hydrochloride or 2-(3,4-dihydro-2,2-dimethyl-6-nitro-2H-1,4-benzoxazin-4-yl)pyridine N-oxide or a salt thereof is a particularly suitable physiologically active substance for increasing the transdermal absorption when used in the composition of the present invention in view of the solubility in aqueous ethanol and the distribution coefficient.

The physiologically active substance used in the transdermal absorption-enhancing agent of the present invention may also be a substance which dissolves or partially dissolves in monoterpenes that constitute the inner oil layer of the preparation of the present invention. These physiologically active substances may be used alone or as a mixture of two or more in an amount sufficient to exert desired drug effects, which may be within the range of from 0.01 to 20% by weight, preferably from 0.1 to 10% by weight, based on the composition of the present invention. The amount of physiologically active substances to be applied is not particularly limited to this range, because it can be controlled by changing the area of the composition to be plastered on the skin.

The composition of the present invention is characterized by the form of emulsion. The O/W-type emulsion type is preferred, but the particle size of the inner oil layer is not limited. With respect to the composition of the present invention, the emulsion form can generally be observed under a microscope, and it should be in a milky white state having a visible light transmittance (at 620 nm) of 10% or less, because higher transmittance means disintegration of emulsion as will be shown later in Test Example 2 and therefore causes considerable decrease in the absorption-enhancing effect.

The external pharmaceutical composition of the emulsion type according to the present invention is prepared by mixing a physiologically active substance wholly or partially soluble in aqueous ethanol, a monoterpene hardly soluble in aqueous ethanol, a nonionic surface active agent, ethanol, water and, if necessary, other components commonly used in emulsion preparations and monoterpenes soluble in aqueous ethanol, in optional order, and then emulsifying the resulting mixture in the usual way. Emulsification may be effected by an ultrasonic treatment or using an equipment in which minute emulsion is obtained through a membrane, such as a microfluidizer (manufactured by MICRO FLUIDICS CORP.) as shown later in Example 12, of which the latter is useful particularly in improving the transdermal penetration.

Though the external pharmaceutical composition of the emulsion type according to the present invention can be used directly as an emulsion preparation, it may also be made into other external preparations such as patches, tapes, cataplasmas, ointments, lotions, solutions, liniments, impregnants,

5

gels, matrices and the like, in the usual way, if necessary, by mixing the aforementioned essential components with pharmaceutically acceptable known components such as an aromatic agent, a stabilizing agent, a moisture keeping agent, an antioxidant, a pH controlling agent, a thickener and the like.

EXAMPLES

The following examples are provided to further illustrate the manufacture of the transdermal absorption preparation of the present invention.

Example 1

One part by weight (to be referred to as "part" hereinafter) of ibusetron hydrochloride (chemical name: (-)-(R)-5-[(1-methyl-1H-indol-3-yl)carbonyl]-4,5,6,7-tetrahydro-1H-benzimidazole hydrochloride) was dissolved in aqueous ethanol (93 parts) consisting of 20 parts of ethanol and 73 parts of phosphate buffer (pH 7.4), and the resulting solution was mixed with 1 part of HCO-60 (trade name, manufactured by Nikko Chemicals; general name, polyoxyethylene hardened castor oil) and 5 parts of Orange Oil (trade name, manufactured by Nakalai Tesque) and then subjected to an ultrasonic treatment (100 W, 4 minutes) using a probe-type ultrasonic generator (SONIFIER 450, manufactured by BRANSON ULTRASONICS CORP.), thereby obtaining an external emulsion.

Examples 2 to 11

In the same manner as described in Example 1, emulsion preparations of Examples 2 to 11 were obtained using respective mixing ratios shown in the formulation table.

Example 12

One part of ibusetron hydrochloride was dissolved in aqueous ethanol (93 parts) consisting of 46 parts of ethanol and 47 parts of phosphate buffer (pH 7.4), and the resulting solution was mixed with 1 part of HCO-60 and 5 parts of Eucalyptus Oil (trade name, manufactured by Nakalai Tesque) and stirred using Automatic Lab-Mixer (trade name, manufactured by Ikeda Rika) to obtain a crude emulsion which was then completely emulsified (treatment pressure: 7,000 to 15,000 psi; time: 5 minutes) using Microfluidizer-M-110F (trade name, manufactured by MICRO FLUIDICS CORP.), thereby obtaining an emulsion preparation.

Examples 13 and 14

In the same manner as described in Example 1, emulsion preparations of Examples 13 and 14 were obtained using respective mixing ratios shown in the formulation table.

Example 15

One part of ibusetron hydrochloride was dissolved in 40 parts of ethanol and 50 parts of purified water, and the resulting solution was mixed with 1 part of HCO-60 and 5 parts of Eucalyptus oil designated by The Pharmacopoeia of Japan (manufactured by Takasago Perfumery Corp.) and then thoroughly stirred using a bath-type ultrasonic generator (Ultrasonic Washer UC-0515, manufactured by Tokyo Choh-onpa Kiki). Then, 2 parts of Aerosil 200 (trade name, manufactured by Aerosil Japan) and 1 part of polyvinylalcohol (B-45) (trade name, manufactured by Denki Kagaku Kogyo) were added, followed by overnight standing and subsequent homogenization (rotation speed: 20,000 rpm; time: 3 minutes) using a homomixer (trade name, Polytron PT10-35; manufactured by Kinematica) to obtain a completely swelled external preparation.

Example 16

One part of ibusetron hydrochloride was dissolved in 40 parts of ethanol and 53 parts of purified water, and the resulting solution was mixed with 1 part of HCO-60 and 5 parts of Eucalyptus oil designated by The Pharmacopoeia of Japan (manufactured by Takasago Perfumery Corp.) and then thoroughly stirred using a bath-type ultrasonic generator. Then, Dackloid NF (trade name, manufactured by Kibun Food Chemifa; general name, propylene glycol alginate), followed by overnight standing, subsequent homogenization using a homomixer, and additional overnight standing to effect complete swelling, thereby obtaining an external preparation.

Examples 17 and 18

In the same manner as described in Example 16, external preparations of Examples 17 and 18 were obtained using respective mixing ratios shown in the formulation table.

Example 19

2-(3,4-Dihydro-2,2-dimethyl-6-nitro-2H-1,4-benzoxazin-4-yl)pyridine N-oxide (to be referred to as Compound A hereinafter) (0.1 part) was dissolved in 49.9 parts of triethylene glycol (manufactured by Nakalai Tesque), and the solution was mixed with 19 parts of phosphate buffer, 25 parts of ethanol, 1 part of HCO-60 and 5 parts of Orange oil and then subjected to ultrasonic treatment (100 W, 4 minutes) using a probe-type ultrasonic generator to obtain an external emulsion preparation.

Example 20

Five parts of tamusulosin hydrochloride was dissolved in aqueous ethanol (89 parts) consisting of 45 parts of ethanol and 44 parts of carmody buffer (pH 7), and the solution was mixed with 1 part of HCO-60 and 5 parts of Eucalyptus oil (manufactured by Nakalai Tesque) and then subjected to ultrasonic treatment (100 W, 4 minutes) using a probe-type ultrasonic generator to obtain an external emulsion preparation.

Examples 21 to 49

In the same manner as described in Example 1, emulsion preparations of Examples 21 to 49 were obtained using respective mixing ratios shown in the formulation table.

Example 50

Gel formulation

| | |
|---|---|
| Tamusulosin hydrochloride | 0.5 |
| Ethanol | 43 |
| Purified water | 49 |
| HCO-60 | 1 |
| Eucalyptus oil designated by The Pharmacopoeia of Japan | 5 |
| Dackloid NF | 1.5 |
| Total | 100 (% by weight) |

(1) Aluminium foil of 3 cm in diameter and 50 μm in thickness was used as a drug-impermeable material, and 200 mg of the above gel formulation was dropped on the foil.
(2) As a support material, porous polypropylene membrane (trade name, Celguard; manufactured by Hoechst) was superposed thereon to seal the gel formulation, thereby obtaining a drug solution-containing material.
(3) The thus formed material was adhered to the central area of a lining material made of a pressure sensitive adhesive plastic foam (manufactured by Marusho) of 5 cm in diameter.
(4) Thereafter, the pressure sensitive adhesive-coated side of the lining material was covered with a release paper to obtain a pharmaceutical preparation.
Component formulations of the transdermal absorption preparations of the present invention obtained in the above examples are shown in the following Tables 1 to 5.

Table 1

| Formulations (Examples 1 to 14) | | | | | | | | | | | | | | |
|---|---|---|---|---|---|---|---|---|---|---|---|---|---|---|
| Components | Example | | | | | | | | | | | | | |
| | 1 | 2 | 3 | 4 | 5 | 6 | 7 | 8 | 9 | 10 | 11 | 12 | 13 | 14 |
| Ibusetron HCl (active ingredient) | 1 | 1 | 1 | 1 | 1 | 1 | 1 | 1 | 1 | 1 | 1 | 1 | 1 | 1 |
| Ethanol | 20 | 30 | 46 | 60 | 65 | 46 | 46 | 46 | 46 | 46 | 46 | 46 | 55 | 60 |
| Phosphate buffer (pH 7.4) | 73 | 63 | 47 | 33 | 28 | 47 | 47 | 47 | 47 | 47 | 47 | 47 | 38 | 33 |
| HCO-60 (surface active agent) | 1 | 1 | 1 | 1 | 1 | 1 | 1 | 1 | 1 | 1 | 1 | 1 | 1 | 1 |
| Terpenes | | | | | | | | | | | | | | |
| Orange oil | 5 | 5 | 5 | 5 | 5 | - | - | - | - | - | - | - | - | - |
| *d*-Limonene | - | - | - | - | - | 5 | - | - | - | - | - | - | - | - |
| Turpentine oil | - | - | - | - | - | - | 5 | - | - | - | - | - | - | - |
| Carene | - | - | - | - | - | - | - | 5 | - | - | - | - | - | - |
| α-Pinene | - | - | - | - | - | - | - | - | 5 | - | - | - | - | - |
| Spearmint oil | - | - | - | - | - | - | - | - | - | 5 | - | - | - | - |
| Eucalyptus oil | - | - | - | - | - | - | - | - | - | - | 5 | 5 | 5 | 5 |
| Total | 100 | 100 | 100 | 100 | 100 | 100 | 100 | 100 | 100 | 100 | 100 | 100 | 100 | 100 |

*d*-Limonene : manufactured by Wako Pure Chemical Industries, Ltd.
Turpentine oil : manufactured by Nakalai Tesque
Carene : manufactured by Aldrich Chemical Company Inc.
α-Pinene : manufactured by Aldrich Chemical Company Inc.
Spearmint oil : manufactured by Givaudan-Roure K.K.
Eucalyptus oil : manufactured by Nakalai Tesque

Table 2

| Formulations (Examples 15 to 20) | | | | | | |
|---|---|---|---|---|---|---|
| Components | Example | | | | | |
| | 15 | 16 | 17 | 18 | 19 | 20 |
| Active ingredients | | | | | | |
| Ibusetron HCl | 1 | 1 | 1 | 1 | - | - |
| Compound A | - | - | - | - | 0.1 | - |
| Tamusulosin HCl | - | - | - | - | - | 5 |
| Ethanol | 40 | 40 | 40 | 40 | 25 | 45 |
| Water | 50 | 53 | 51 | 49 | - | - |
| Phosphate buffer (pH) | - | - | - | - | 19 (7) | - |
| Carmody buffer (pH) | - | - | - | - | - | 44 (7) |
| HCO-60 (surface active agent) | 1 | 1 | 1 | 1 | 1 | 1 |
| Orange oil | - | - | - | - | 5 | - |
| Eucalyptus oil | 5 | 5 | 5 | 5 | - | 5 |
| Triethylene glycol (solubilizing aid) | - | - | - | - | 49.9 | - |
| Thickeners | | | | | | |
| Aerosil 200 | 2 | - | - | - | - | - |
| Polyvinylalcohol B-45 | 1 | - | - | - | - | - |
| Dackloid NF | - | 1 | 2 | 4 | - | - |
| Total | 100 | 100 | 100 | 100 | 100 | 100 |

Table 3  Formulations (Examples 21 to 35)

| Compo-nents | Example | | | | | | | | | | | | | | |
|---|---|---|---|---|---|---|---|---|---|---|---|---|---|---|---|
| | 21 | 22 | 23 | 24 | 25 | 26 | 27 | 28 | 29 | 30 | 31 | 32 | 33 | 34 | 35 |
| Ibusetron HCl (active ingredient) | 1 | 1 | 1 | 1 | 1 | 1 | 1 | 1 | 1 | 1 | 1 | 1 | 1 | 1 | 1 |
| Ethanol | 46 | 46 | 46 | 46 | 46 | 46 | 46 | 10 | 10 | 10 | 10 | 10 | 10 | 10 | 10 |
| Phosphate buffer (pH 7.4) | 47 | 47 | 47 | 47 | 47 | 47 | 47 | 83 | 83 | 83 | 83 | 83 | 83 | 83 | 83 |
| Orange oil | 5 | 5 | 5 | 5 | 5 | 5 | 5 | 5 | 5 | 5 | 5 | 5 | 5 | 5 | 5 |
| Surface active agent | | | | | | | | | | | | | | | |
| HCO-10 | — | — | — | — | 0.3 | — | — | — | — | — | — | — | — | — | — |
| HCO-20 | — | — | — | — | — | — | — | — | — | — | — | 1 | — | — | — |
| HCO-30 | 1 | — | — | — | — | — | | — | — | — | — | — | — | — | — |
| HCO-40 | — | 1 | — | — | — | — | — | — | — | — | — | — | — | — | — |
| HCO-50 | — | — | 1 | — | — | — | — | — | — | — | — | — | — | — | — |
| HCO-60 | — | — | — | 1 | 0.7 | 0.6 | 0.5 | — | — | — | — | — | — | — | — |
| BL-9EX | — | — | — | — | — | — | — | — | 1 | — | — | — | — | — | — |
| MYS-25 | — | — | — | — | — | — | — | — | — | 1 | — | — | — | — | — |
| MYS-40 | — | — | — | — | — | — | — | — | — | — | — | — | 1 | — | — |
| Tween 20 | — | — | — | — | — | — | — | — | — | — | 1 | — | — | — | — |
| Tween 40 | — | — | — | — | — | — | — | — | — | — | — | — | — | — | 0.5 |
| Tween 65 | — | — | — | — | — | — | — | — | — | — | — | — | — | 1 | — |
| Tween 80 | — | — | — | — | — | — | 0.5 | 1 | — | — | — | — | — | — | — |
| Span 20 | — | — | — | — | — | 0.4 | — | — | — | — | — | — | — | — | — |
| Span 80 | — | — | — | — | — | — | — | — | — | — | — | — | — | — | 0.5 |
| Total | 100 | 100 | 100 | 100 | 100 | 100 | 100 | 100 | 100 | 100 | 100 | 100 | 100 | 100 | 100 |

Annexed Table: General names and chemical compositions of the surfactants shown in Table 3 (all manufactured by Nikko Chemicals)

|  | General name | Chemical composition* |
|---|---|---|
| HCO-10 | polyoxyethylene hardened castor oil | POE (10) hardened castor oil |
| HCO-20 | " | POE (20) hardened castor oil |
| HCO-30 | " | POE (30) hardened castor oil |
| HCO-40 | " | POE (40) hardened castor oil |
| HCO-50 | " | POE (50) hardened castor oil |
| HCO-60 | " | POE (60) hardened castor oil |
| BL-9EX | polyoxyethylene alkyl ether | POE (9) lauryl ether |
| MYS-25 | polyethylene glycol fatty acid ester | POE (25) monostearate |
| MYS-40 | " | POE (40) monostearate |
| Tween 20 | polyoxyethylene sorbitan fatty acid ester | POE (20) sorbitan monolaurate |
| Tween 40 | " | POE (20) sorbitan monopalmitate |
| Tween 60 | " | POE (20) sorbitan monostearate |
| Tween 65 | " | POE (20) sorbitan tristearate |
| Tween 80 | " | POE (20) sorbitan monooleate |
| Span 20 | sorbitan fatty acid ester | sorbitan monolaurate |
| Span 80 | " | sorbitan monooleate |

*: POE means polyoxyethylene, and each figure in ( ) indicates addition polymerization degree of oxidized ethylene.

Table 4  Formulations (Examples 36 to 45)

| Components | Example | | | | | | | | | |
|---|---|---|---|---|---|---|---|---|---|---|
| | 36 | 37 | 38 | 39 | 40 | 41 | 42 | 43 | 44 | 45 |
| Ibusetron HCl (active ingredient) | 1 | 1 | 1 | 1 | 1 | 1 | 1 | 1 | 1 | 1 |
| Ethanol | 45 | 45 | 30 | 45.1 | 45 | 45 | 45 | 45 | 45 | 45 |
| Phosphate buffer (pH 7.4) | 47 | 46 | 61 | 47 | 46 | 46 | 46 | 47 | 47 | 46 |
| HCO-60 (surface active agent) | 1 | 1 | 1 | 1 | 1 | 1 | 1 | 1 | 1 | 1 |
| Terpenes | | | | | | | | | | |
| Orange oil | 5 | 5 | 5 | 5 | 5 | 5 | 5 | 5 | — | — |
| Eucalyptus oil | — | — | — | — | 2 | — | — | — | 5 | 5 |
| Mentha oil | — | 2 | 2 | — | — | — | — | — | — | 2 |
| *l*-Menthol | 1 | — | — | — | — | — | — | — | 1 | — |
| Terpineol | — | — | — | 0.9 | — | — | — | — | — | — |
| Cineole | — | — | — | — | — | 2 | — | — | — | — |
| *l*-Carvone | — | — | — | — | — | — | 2 | — | — | — |
| Linalool | — | — | — | — | — | — | — | 1 | — | — |
| Total | 100 | 100 | 100 | 100 | 100 | 100 | 100 | 100 | 100 | 100 |

Mentha oil     : manufactured by Kosakai Seiyaku

*l*-Menthol     : manufactured by Nakalai Tesque

Terpineol     : manufactured by Tokyo Kasei Kogyo

Cineole     : manufactured by Nakalai Tesque

*l*-Carvone     : manufactured by Kanto Chemical

Linalool     : manufactured by Nakalai Tesque

Eucalyptus oil: manufactured by Nakalai Tesque

Table 5

| Formulations (Examples 46 to 49) | | | | |
|---|---|---|---|---|
| Components | Example | | | |
| | 46 | 47 | 48 | 49 |
| Active ingredient | | | | |
| Ibusetron hydrochloride | 1 | - | - | - |
| Nicardipine hydrochloride | - | 1 | - | - |
| Indometacin | - | - | 1 | - |
| Tolnaftate | - | - | - | 0.5 |
| Ethanol | 35 | 45 | 45 | 45 |
| Phosphate buffer (pH) | 55.95 (7.4) | 46 (7.1) | 46 (7.0) | 46.5 (7.0) |
| HCO-60 (surfactant) | 1 | 1 | 1 | 1 |
| Orange oil | 5 | 5 | 5 | 5 |
| Mentha oil | 2 | 2 | 2 | 2 |
| Xanthan gum (thickener) | 0.05 | - | - | - |
| Total | 100 | 100 | 100 | 100 |
| Xanthan gum: general name; manufactured by San-ei Chemical Industries under a trade name of Sun Ace | | | | |

Effects of the Invention

Since the external pharmaceutical composition of the emulsion type according to the present invention markedly improves the effect of terpenes to enhance the transdermal absorption of physiologically active substances which are wholly or partially soluble in aqueous ethanol and renders possible production of practical TTS preparations, it is useful in terms of its excellent characteristics in that it can achieve drug absorption which is necessary for precisely expressing drug effects under precise administration control and that it does not irritate the skin. In addition, the composition of the present invention itself is useful not only as an external emulsion preparation having an excellent transdermal absorption property, but also as a composition for the transdermal absorption pharmaceutical preparations having an excellent transdermal absorption property, because it can be applied to the conventional external preparations such as plasters, ointments such as gel ointments, and solutions, and the like, without spoiling its emulsion form.

The excellent transdermal absorption-enhancing effect of the pharmaceutical preparation of the present invention and the state of the emulsion preparation were confirmed by the following methods (evaluation of the results of each test will be described later in the column "Summary").

Test Methods

Test Example 1: Effects of the pharmaceutical preparation of the present invention in the state of the emulsion preparation on the effect of a terpene to enhance the transdermal drug absorption

[Preparation method]

Using the aforementioned component formulation of Example 3 (see Table 1), an emulsion preparation was prepared in accordance with the procedure of Example 1. Also, a control solution (Control Example 1) was prepared by repeating the procedure of Example 1 except that the emulsification step was not employed.

[Test method]

Using the skin of Yucatan microswine, the transdermal absorption was evaluated by an *in vitro* skin penetration test.

The dorsal skin of Yucatan microswine was attached to a Franz-type diffusion cell (effective cell area: 0.7 cm$^2$), a sample was added to the donor side, an isotonic solution of 10 mM potassium dihydrogenphosphate was added to the receptor side and then the test was carried out at a cell temperature of 37 °C. Whole volume of the receptor sample was collected 2, 4, 8 and 24 hours thereafter, and the concentration of ibusetron hydrochloride penetrated through the skin was measured by the liquid chromatography method.

[Results]

Table 6

|  | Flux ($\mu$g/cm$^2$/hr) |
|---|---|
| Example 3 | 24.8 |
| Control Example 1 | 7.2 |

Test Example 2: Effects of ethanol content on the transdermal absorption-enhancing effect and emulsification state of the pharmaceutical preparation of the present invention

[Preparation method]

Using the aforementioned component formulations of Examples 1 to 5 (see Table 1), emulsion preparations were prepared in accordance with the procedure of Example 1. Also, a control solution (Control Example 2) in which ethanol was not used and solutions showing no emulsified state (Comparative Examples 1 and 2) were prepared in the same manner using respective component formulations shown in the following table.

Table 7

| Control- or Comparative- Example No. / Components | Control Example 2 | Comparative Example 1 | Comparative Example 2 |
|---|---|---|---|
| Ibusetron hydrochloride (active ingredient) | 1 | 1 | 1 |
| Ethanol | 0 | 70 | 83 |
| Phosphate buffer (pH 7.4) | 93 | 23 | 10 |
| HCO-60 (surface active agent) | 1 | 1 | 1 |
| Orange oil (terpene) | 5 | 5 | 5 |
| Total | 100 | 100 | 100 |

[Test method]

The test was carried out in accordance with the procedure of Test Example 1.

[Results]

Table 8

| | Control Example 2 | Example | | | | | Comparative Example | |
|---|---|---|---|---|---|---|---|---|
| | | 1 | 2 | 3 | 4 | 5 | 1 | 2 |
| Flux ($\mu$g/cm$^2$/hr) | 1.65 | 3.37 | 22.7 | 24.8 | 38.5 | 50.2 | 0.59 | 0.08 |
| Appearance of preparation | Emulsion | Emulsion | Emulsion | Emulsion | Emulsion | Emulsion | Semi-transparent | Semi-transparent |
| Penetration ratio | 0 | 0 | 0 | 0 | 0 | 0.02 | 12.05 | 54.75 |

Test Example 3: Effects of the use of other oil terpenes which form the inner oil layer in the pharmaceutical preparation of the present invention on the transdermal absorption-enhancing effect

[Preparation method]

Using the aforementioned component formulations of Examples 6 to 16 (see Tables 1 and 2), emulsion preparations were prepared in accordance with the procedure of Example 1.

[Test method]

The test was carried out in accordance with the procedure of Test Example 1.

[Results]

Table 9

| Example No. | 6 | 7 | 8 | 10 | 11 | 12 | 13 | 14 | 15 | 16 |
|---|---|---|---|---|---|---|---|---|---|---|
| Flux ($\mu$g/cm$^2$/hr) | 27.1 | 12.2 | 15.7 | 23.8 | 19.3 | 30.8 | 32.9 | 32.1 | 41.9 | 22.8 |

Test Example 4: Effects of the sole use of a monoterpene hardly soluble in aqueous ethanol on the transdermal absorption-enhancing effect (examination using other drug)

[Preparation method]

Using the aforementioned component formulations of Examples 19 and 20 (see Table 2), emulsion preparations were prepared in accordance with the procedure of Example 1.
As a control (Control Example 3), a formulation was prepared by excluding ethanol, HCO-60 and Orange oil from the system used in Example 19.

[Test method]

The test was carried out in accordance with the procedure of Test Example 1.

[Results]

| Example No. | Control Example 3 | Example 19 | Example 20 |
|---|---|---|---|
| Flux ($\mu$g/cm$^2$/hr) | 0.047 | 1.99 | 96.2 |

Test Example 5: Effects of various surface active agents on the state of the pharmaceutical preparation of the present invention as emulsion preparations

[Preparation method]

Using the aforementioned component formulations of Examples 21 to 35 (see Table 3), an attempt was made to prepare emulsion preparations in accordance with the procedure of Example 1 in order to examine the presence or absence of emulsification.

17

[Results]

Emulsification was confirmed with respect to all preparations of Examples 21 to 35.

In this instance, emulsification was not found when HCO-10 alone was used as the source of the component formulation of Example 25 (cf. Table 3). Since the HLB value of HCO-10 is about 6.5 and the ethanol content was 46% of the total composition, it was suggested that the type of surface active agent and the ethanol content should properly be selected.

Test Example 6: Transdermal absorption-enhancing effect when monoterpenes hardly soluble and wholly soluble in aqueous ethanol were used in combination (1) (comparison with a case of the sole use of a monoterpene hardly soluble in aqueous ethanol)

[Preparation method]

Using the aforementioned component formulations of Examples 36 to 45 (see Table 4), emulsion preparations were prepared in accordance with the procedure of Example 1. As controls, preparations having the component formulations of Examples 3 and 11 (cf. Table 1) in which a monoterpene hardly soluble in aqueous ethanol (Orange oil or Eucalyptus oil) was used alone were prepared (Control Examples 4 and 5).

[Test method]

The test was carried out in accordance with the procedure of Test Example 1.

[Results]

Table 10

|  | Examples | | | | | | | |
|---|---|---|---|---|---|---|---|---|
|  | 36 | 37 | 39 | 41 | 42 | 43 | 44 | 45 |
| Flux ($\mu$g/cm$^2$/hr) | 39.7 | 46.8 | 41.0 | 26.4 | 43.1 | 48.8 | 43.9 | 30.1 |
|  | Control Example 4 (Example 3) | | | Control Example 5 (Example 11) | | | | |
| Flux ($\mu$g/cm$^2$/hr) | 24.8 | | | 19.3 | | | | |

Test Example 7: Transdermal absorption-enhancing effect when monoterpenes hardly soluble and wholly soluble in aqueous ethanol were used in combination (2) (examination using other drug)

[Preparation method]

Using the aforementioned component formulations of Examples 47 and 48 (see Table 5), emulsion preparations were prepared in accordance with the procedure of Example 1 (except that nicardipine hydrochloride or indometacin was used as the drug instead of ibusetron hydrochloride).

As a control, a formulation was prepared by excluding Orange oil and Mentha oil from the system used in Example 47 (Control Example 6).

[Test method]

The test was carried out in accordance with the procedure of Test Example 1.

[Results]

Table 11

|  | Control Example 6 | Example 47 | Example 48 |
|---|---|---|---|
| Drug | Nicardipine HCl | Nicardipine HCl | Indometacin |
| Flux ($\mu$g/cm$^2$/hr) | 0.01 | 2.73 | 39.94 |

Test Example 8: Skin-irritability of the emulsion preparation of the present invention

[Preparation method]

An emulsion preparation having a component formulation shown in the following table was prepared in accordance with the procedure of Example 1. A control solution (Control Example 7) was also prepared by excluding ethanol from the formulation.

Table 12

|  | Control Example 7 | Test Example 1 |
|---|---|---|
| Ethanol | 0 | 30 |
| Phosphate buffer (pH 7.4) | 94 | 64 |
| HCO-60 | 1 | 1 |
| Orange oil | 5 | 5 |
| Total | 100 | 100 |

[Test method]

A patch test was carried out in the following manner.

An adhesive plaster for the patch test (manufactured by Torii Yakuhin, 18 mm in diameter) was impregnated with 50 $\mu$l of each sample and applied to the upper arm of each of three healthy males.

The plaster was removed 24 hours thereafter to evaluate its irritability.

[Results]

In the case of Test Example 1, a slightly smart feeling to the skin was observed at early stage of the application, but erythema after removal of the plaster was found slightly only in one of the three volunteers.

In the case of Control Example 7, on the other hand, all of the three volunteers complained of severe pain immediately after the application so that the plasters had to be removed within 1 hour. In addition, strong erythema and slight edema were observed after the removal.

[Summary]

The following describes the results obtained in Test Examples 1 to 8. As described in the foregoing, ibusetron hydrochloride was blended in the pharmaceutical preparation of the present invention as the active ingredient (medicine) in Test Examples 1 to 3, and tamusulosin hydrochloride was blended in the pharmaceutical preparation of the present invention instead of the former, in Test Example 4.

Ibusetron hydrochloride was blended in the pharmaceutical preparation of the present invention as the active ingredient (medicine) in Test Examples 5 and 6, and nicardipine hydrochloride or indometacin was blended in Test Example 7 instead of the former.

In Test Example 8, skin-irritability of the emulsion preparation of the present invention was tested.

At first, the results of Test Example 1 showed that the pharmaceutical preparation of the present invention as an of emulsion preparation markedly increases the transdermal absorption-enhancing effect of a terpene (Orange oil) in comparison with the non-emulsified control.

In Test Example 2, it was revealed that the characteristic features of the pharmaceutical preparation of the present invention, namely blending of ethanol which is effective as a transdermal absorption-enhancing auxiliary in an amount of 20 to 65 parts and the state of the preparation as a form of emulsion, are effective in synergistically increasing the transdermal absorption-enhancing effect of terpenes, when compared with the emulsion preparation in which ethanol was not added (Control Example 2) and with the solutions which contained ethanol but showed no state as an emulsion preparation (Comparative Examples 1 and 2).

In Test Example 3, the examination was carried out on the transdermal absorption-enhancing effect of various oil terpenes which form the inner oil layer due to their hard solubility in aqueous ethanol, which revealed that the pharmaceutical preparation of the present invention can exert excellent transdermal absorption effect by the use of any of these terpenes. Markedly excellent effect was obtained particularly in a system in which *d*-limonene, Spearmint oil, Eucalyptus oil, or the like was used. In addition, it was found by this test that the emulsion preparation shows the most excellent transdermal absorption effect when completely emulsified using Vortex Mixer and Microfluidizer (Example 12).

Based on this information, the transdermal absorption of various drugs was examined in Test Example 4, by blending Compound A or tamusulosin hydrochloride instead of ibusetron hydrochloride.

As a result, the pharmaceutical preparation of the present invention also showed excellent transdermal absorption when these drugs were blended, thus confirming that the pharmaceutical preparation of the present invention is useful in improving transdermal absorption of not only ibusetron hydrochloride but also other various drugs.

In this connection, as is evident from the data of Control Example 3 and Example 19, Compound A can hardly be absorbed through the skin by the conventional drug preparation techniques, but its transdermal absorption can be improved considerably by the emulsion preparation of the present invention.

A feature of the pharmaceutical preparation of the present invention, namely a property to maintain the state of the emulsion preparation in spite of the blending of ethanol which is generally known to have a function to break the emulsion, was achieved by blending a specified amount of ethanol with a nonionic surface active agent such as HCO-60 or the like, so that the presence or absence of emulsification was examined in Test Example 5 using various nonionic surface active agents. As a result, the emulsification was observed in all systems tested, thus showing a fact that various nonionic surface active agents can be applied to the pharmaceutical preparation of the present invention, which is advantageous from the viewpoint of the production of pharmaceutical preparations.

In Test Examples 6 and 7, the transdermal absorption effect was examined when terpenes hardly soluble and wholly soluble in aqueous ethanol were used in combination.

In Test Example 6, a system in which terpenes were used in combination showed more excellent transdermal absorption in comparison with a control in which a terpene hardly soluble in aqueous ethanol (Orange oil or Eucalyptus oil) was used alone, and particularly excellent results were obtained by a combination of Orange oil with Mentha oil, a combination of Orange oil with *l*-menthol, a combination of Orange oil with linalool, a combination of Orange oil with terpineol, a combination of Orange oil with carvone, a combination of Eucalyptus oil with Mentha oil and a combination of Eucalyptus oil with *l*-menthol.

Based on this information, effects of the combined use of terpenes on the transdermal absorption were further examined in Test Example 7 by blending nicardipine hydrochloride or indometacin, instead of ibusetron hydrochloride, in a system in which Orange oil and Mentha oil were used in combination.

As a result, the pharmaceutical preparation of the present invention also showed excellent transdermal absorption when these drugs were blended, thus confirming that the pharmaceutical preparation of the present invention is useful in improving transdermal absorption of not only ibusetron hydrochloride but also other various drugs.

In this connection, as is evident from the data of Control Example 6 and Example 47, nicardipine hydrochloride can hardly be absorbed through the skin by the conventional drug preparation techniques, but the transdermal absorption can be improved considerably by the emulsion preparation of the present invention.

Finally, when irritability of the emulsion formulation of the present invention to the human skin was examined in Test Example 8, it was found completely unexpectedly that the skin irritability was hardly observed in spite of the blending of ethanol which has been reported as a skin irritant (Abstract of Papers, the 6th Annual Meeting of the Pharmaceutical Society of Japan, pp. 40 - 41, 1990).

On the basis of the above results, it was confirmed that the preparation of the present invention considerably improves the effect of terpenes to enhance the transdermal absorption of drugs and does not irritate the skin.

INDUSTRIAL APPLICABILITY

According to the pharmaceutical preparation (emulsion) of the present invention, the skin penetration of drugs generally considered to have a relatively low skin penetrating ability can be improved sharply, when an emulsion preparation is produced by blending ethanol with a monoterpene as a transdermal absorption-enhancing agent and adding a nonionic surface active agent to the resulting blend.

Conventional pharmaceutical preparations containing no ethanol cannot show sufficient skin penetration of drugs and are practically disadvantageous from the viewpoint of skin irritability when limonene is used.

The inventors of the present invention have found unexpectedly that these problems can be resolved when ethanol generally considered to be difficult in producing emulsion preparations because of its function to dissolve monoterpenes is blended with a specified nonionic surface active agent, and have accomplished the pharmaceutically stable emulsion preparation of the present invention.

As described in the aforementioned Examples, it was confirmed that the present invention can provide sufficient skin penetration of any drug which dissolves wholly or partially in aqueous ethanol. In consequence, the inventive drug preparation production technique does not depend on the physical properties of drugs and therefore is applicable to various purposes.

**Claims**

1.  An external pharmaceutical composition of O/W emulsion type, which comprises (a) a physiologically active substance wholly or partially soluble in aqueous ethanol, (b) a monoterpene hardly soluble in aqueous ethanol or a mixture thereof with a monoterpene soluble in aqueous ethanol, (c) a nonionic surface active agent, and (d) aqueous ethanol.

2.  The external pharmaceutical composition according to claim 1, wherein said monoterpene is at least one compound selected from essential oils containing a cyclic monoterpene, and cyclic monoterpenes belonging to hydrocarbons.

3.  The external pharmaceutical composition according to claim 2, wherein said essential oils containing a cyclic monoterpene or cyclic monoterpenes belonging to hydrocarbons are selected from at least one of limonene, Orange oil, Eucalyptus oil, Turpentine oil, Lemon oil and Spearmint oil and blended in an amount of from 1% by weight to 50% by weight based on the total components.

4.  The external pharmaceutical composition according to claim 3, wherein said essential oil containing a cyclic monoterpene is Eucalyptus oil.

5.  The external pharmaceutical composition according to claim 1, wherein said mixture of a monoterpene hardly soluble in aqueous ethanol with a monoterpene soluble in aqueous ethanol is selected from the combination consisting of Orange oil with Mentha oil, Orange oil with *l*-menthol, Orange oil with linalool, Eucalyptus oil with Mentha oil and Eucalyptus oil with *l*-menthol.

6.  The external pharmaceutical composition according to claim 1, wherein said nonionic surface active agent is polyoxyethylene hardened castor oil.

7.  The external pharmaceutical composition according to claim 1, wherein ethanol is blended in an amount of from 5% by weight to 50% by weight based on the total components.

8.  The external pharmaceutical composition according to claim 1, wherein said physiologically active substance wholly or partially soluble in aqueous ethanol is selected from ibusetron hydrochloride, tamusulosin hydrochloride and 2-(3,4-dihydro-2,2-dimethyl-6-nitro-2H-1,4-benzoxazin-4-yl)pyridine N-oxide or salts thereof.

9. The external pharmaceutical composition according to claim 1, which is used as patches.

| INTERNATIONAL SEARCH REPORT | International application No. |
|---|---|
| | PCT/JP94/00066 |

**A. CLASSIFICATION OF SUBJECT MATTER**

Int. Cl$^5$ A61K9/107, 9/70, A61K47/06

According to International Patent Classification (IPC) or to both national classification and IPC

**B. FIELDS SEARCHED**

Minimum documentation searched (classification system followed by classification symbols)

Int. Cl$^5$ A61K9/10-9/107, 9/70, A61K47/06-47/14

Documentation searched other than minimum documentation to the extent that such documents are included in the fields searched

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| Y | JP, A, 4-193826 (Shirogane Seiyaku K.K.), July 13, 1992 (13. 07. 92), Claims 1, 2, lines 1 to 6, upper left column, page 2, line 17, lower right column, page 2 to line 5, upper left column, page 3, lines 11 to 15, lower right column, page 3, (Family: none) | 1-4 |
| Y | JP, A, 3-173816 (Yamanouchi Pharmaceutical Co., Ltd.), July 29, 1991 (29. 07. 91), Claims 1 to 3, lines 9 to 18, upper left column, lines 14 to 16, upper right column, line 17, lower left column to line 8, lower right column, page 3, (Family: none) | 1-7 |
| Y | JP, A, 2-207024 (FSK K.K.), August 16, 1990 (16. 08. 90), Claim, lines 13 to 18, lower left column, lines 4 to 7, lower right column, page 3, lines 2 to 9, upper left column, page 4, (Family: none) | 1-3, 9 |

☐ Further documents are listed in the continuation of Box C.     ☐ See patent family annex.

| | |
|---|---|
| * Special categories of cited documents:<br>"A" document defining the general state of the art which is not considered to be of particular relevance<br>"E" earlier document but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| April 11, 1994 (11. 04. 94) | May 10, 1994 (10. 05. 94) |

| Name and mailing address of the ISA/ | Authorized officer |
|---|---|
| Japanese Patent Office | |
| Facsimile No. | Telephone No. |

Form PCT/ISA/210 (second sheet) (July 1992)